(19) Europäisches Patentamt
European Patent Office
Office européen des brevets

(11) EP 0 507 337 B1

(12) **EUROPÄISCHE PATENTSCHRIFT**

(45) Veröffentlichungstag und Bekanntmachung des Hinweises auf die Patenterteilung:
**23.10.1996 Patentblatt 1996/43**

(21) Anmeldenummer: **92105822.8**

(22) Anmeldetag: **03.04.1992**

(51) Int Cl.[6]: **C07F 9/113**, A61K 31/66, C07F 9/572, C07F 9/59, C07F 9/10, C07F 9/173, C07F 9/655, C07F 9/09

(54) **Neue Erucyl-, Brassidyl- und Nervonylderivate**

Novel erucyl, brassidyl and nervonyl derivatives

Nouveaus dérivés dérucyle, brassidyle et nervonyle

(84) Benannte Vertragsstaaten:
**AT BE CH DE DK ES FR GB GR IT LI LU MC NL PT SE**

(30) Priorität: **05.04.1991 DE 4111105**

(43) Veröffentlichungstag der Anmeldung:
**07.10.1992 Patentblatt 1992/41**

(73) Patentinhaber: **Max-Planck-Gesellschaft zur Förderung der Wissenschaften e.V.**
**D-37073 Göttingen (DE)**

(72) Erfinder: **Eibl, Hansjörg, Prof. Dr.**
**W-3406 Bovenden-Eddigehausen (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**81635 München (DE)**

(56) Entgegenhaltungen:
**EP-A- 0 061 872** **EP-A- 0 108 565**
**US-A- 4 562 005**

- **CANCER CHEMOTHERAPY AND PHARMACOLOGY Bb. 30, Nr.2, 1992, Seiten 105-112 J. KÖTTING.**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 507 337 B1

## Beschreibung

Die vorliegende Erfindung betrifft neue Phospholipide, die einen Erucyl-, Brassidyl- oder Nervonyl-Rest enthalten, und deren Verwendung als Arzneimittel, insbesondere zur Bekämpfung von Tumoren sowie Protozoen- und Pilzerkrankungen, sowie zur Therapie von Autoimmunerkrankungen und Knochenmarkschädigungen.

Die Verwendung von Phospholipiden als Arzneimittel ist bekannt. Die EP-A 0 108 565 offenbart Phospholipide, einschließlich ihrer pharmazeutisch verträglichen Salze der allgemeinen Formel

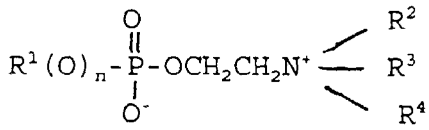

worin $R^1$ ein aliphatischer $C_8$-$C_{30}$-Kohlenwasserstoffrest ist, $R^2$, $R^3$ und $R^4$ unabhängig Wasserstoff oder $C_1$-$C_5$-Alkyl sind oder worin

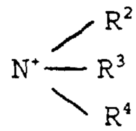

cyclisches Ammonium darstellt und n 0 oder 1 ist. Vorzugsweise ist $R^1$ ein aliphatischer Kohlenwasserstoffrest mit 12 bis 22, besonders bevorzugt mit 14, 17 oder 18 Kohlenstoffatomen. Diese Verbindungen werden als geeignet zur Bekämpfung von Tumorzellen oder Pilzerkrankungen oder zur Verwendung als Pflanzenschutzmittel bezeichnet.

Die EP-A-0 061 872 offenbart Phospholipide mit einer ähnlichen allgemeinen Formel wie der vorstehend gezeigten, wobei die Gruppe $R^1(O)_n$- durch die Gruppe $R^1$-O-$(CH_2)_2$-O- ersetzt ist, $R^1$ ein gegebenenfalls substituierter aliphatischer $C_8$-$C_{26}$ Kohlenwasserstoffrest ist und $R^2$, $R^3$ und $R^4$ dieselbe Bedeutung haben wie in der EP-A-0 108 565, jedoch auch substituiert sein können. Bevorzugt ist $R^1$ $C_{10}$-$C_{20}$-Alkyl, $C_{10}$-$C_{20}$-Alkenyl oder $C_{14}$-$C_{24}$-Aralkyl. Die Wirkung dieser Verbindungen wird als antitumoriell sowie fungizid bzw. antimykotisch beschrieben.

Die DE-OS 32 39 817 offenbart Glycerinderivate der allgemeinen Formel

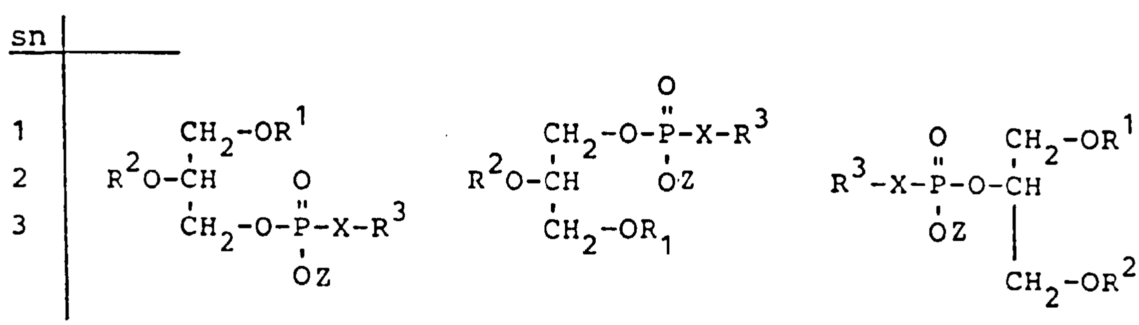

worin $R^1$ und $R^2$ u.a. substituierte oder unsubstituierte Alkylreste mit 1 bis 24 Kohlenstoffatomen bedeuten können, X u.a. Sauerstoff bedeuten kann und $R^3$ u.a. eine Aminoalkylgruppe und eine N-Alkylaminoalkylgruppe mit 2 bis 14 Kohlenstoffatomen in den Alkylresten bedeuten kann. Weiterhin sind dort auch allgemeine Verfahren zur Herstellung der obigen Verbindungen offenbart, so daß man Glycerinderivate mit verschiedenen Resten stellungsspezifisch und mit hoher Selektivität herstellen kann.

Die DE-OS 36 41 379 offenbart Verbindungen der allgemeinen Formel

$$R\text{-}Y\text{-}PO_2^{\ominus}\text{-}X\text{-}R^1$$

worin R einen Kohlenwasserstoffrest mit 12 bis 24 C-Atomen sein kann, X u.a. O bedeutet und $R^1$ u.a. eine Alkylgruppe bedeuten kann, die mit unterschiedlichen Aminogruppen substituiert sein kann. Vorzugsweise ist R ein Alkyl- oder Alkylenrest mit 14 bis 20 C-Atomen, X = O und $R^1$ = Trialkylammoniumethyl mit 1 bis 3 C-Atomen je Alkylgruppe. Besonders bevorzugt sind die Verbindungen Hexadecylphosphocholin und Oleylphosphocholin. Ferner wird die Verwendung der oben genannten Verbindungen als Arzneimittel, insbesondere zur Behandlung von Tumoren offenbart. Derartige Arzneimittel können auch zur topischen Behandlung von Hauttumoren als zusätzlichen Wirkstoff Alkylglycerin enthalten. Es werden auch allgemeine Herstellungsmethoden für die Verbindungen gemäß der oben genannten allgemeinen Formel gelehrt.

Die DE-OS 36 41 491 offenbart ein Antitumor-wirksames Arzneimittel, das Hexadecylphosphocholin als Wirkstoff sowie gegebenenfalls weitere übliche pharmazeutische Zusatz-, Träger- und/oder Verdünnungsstoffe enthält. Ein derartiges Arzneimittel kann als zusätzlichen Wirkstoff auch ein Alkylglycerin enthalten.

Weiterhin ist bekannt, daß ein in Position 2 methyliertes Glycerinderivat von Hexadecylphosphocholin das Etherphospholipid ET-18-$OCH_3$ ein sehr wirksames Antitumormittel ist (siehe z.B. Berdel et al. in Phospholipids and Cellular Regulation (1985), herausgegeben J.F. Kuo, Seiten 41-74, CRC-Press, Boca Raton, Florida). Ferner hat sich ET-18-$OCH_3$ auch als geeignetes Arzneimittel zur Bekämpfung von Autoimmunkrankheiten wie etwa Multipler Sklerose erwiesen.

Aus den oben genannten Veröffentlichungen ist ersichtlich, daß Phosphatidylamine und Etherlysolecitine als Arzneimittel für unterschiedliche Anwendungen, z.B. zur Bekämpfung von Tumoren und Autoimmunerkrankungen, geeignet sind. Als bisher wirksamste Arzneimittel aus dieser Verbindungsklasse haben sich Hexadecylphosphocholin und ET-18-$OCH_3$ erwiesen. Jedoch auch diese Substanzen haben den Nachteil, daß sie bei hoher Dosierung eine toxische Wirkung zeigen. Ein weiterer Nachteil der bisher bekannten Phospholipide ist, daß sie eine Zytolyse von Zellen, insbesondere ein Hämolyse von Erythrozyten bewirken. Deshalb können diese Substanzen nicht intravenös appliziert werden, da dies zu hämolytischen und gewebenekrotischen Begleiterscheinungen führt, wie detailliert in der DE 40 26 136 beschrieben ist.

Aufgabe der vorliegenden Erfindung war es somit, neue Phospholipide zu finden, die als Arzneimittel wirksam sind, bei denen jedoch die Nachteile des Standes der Technik mindestens teilweise beseitigt sind.

Die erfindungsgemäße Aufgabe wird gelöst durch Verbindungen der allgemeinen Formel I

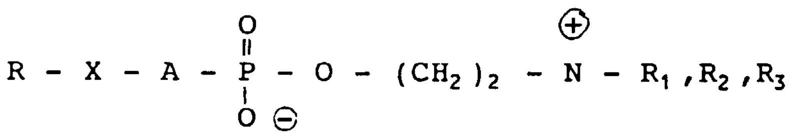

(I)

in der R einen Erucyl-, Brassidyl- oder Nervonyl-Rest,

$R_1$, $R_2$ und $R_3$ unabhängig voneinander je einen geradkettigen, verzweigten, cyclischen gesättigten oder ungesättigten Alkylrest mit 1 bis 4 C-Atomen, der auch eine OH-Gruppe enthalten kann und wobei zwei dieser Reste auch zu einem Ring verbunden sein können,

A eine Einfachbindung oder eine der Gruppen mit den Formeln

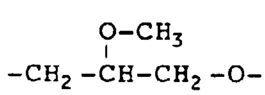

(II),

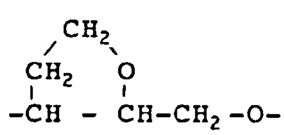

(III),

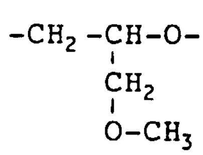

(IV),

$$-CH_2-CH_2-CH_2-O- \qquad (V),$$

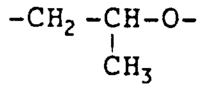

(VI)

oder

$$-CH_2-CH_2-O \qquad (VII)$$

ist,
wobei die Gruppen (II) bis (VII) eine solche Orientierung besitzen, daß das Sauerstoffatom an das Phosphoratom der Verbindung (I) gebunden ist,
und X ein Sauerstoffatom ist, wenn A eine Einfachbindung ist oder ein Sauerstoff- oder Schwefelatom bedeutet, wenn A eine der Gruppen (II) bis (VII) ist.

In der obigen Formel (I) bedeutet X vorzugsweise ein Sauerstoffatom, und A bedeutet vorzugsweise eine Einfachbindung. Weiterhin bevorzugt ist, daß $R_1$, $R_2$ und $R_3$ jeweils Methylgruppen sind, so daß es sich bei den resultierenden Verbindungen um Phosphocholinderivate handelt. Besonders bevorzugt ist Erucylphosphocholin.

Erucyl-, Brassidyl- und Nervonyl-Reste sind langkettige Alkylreste. Sie können aus den entsprechenden Fettsäuren, die teilweise natürlich vorkommen, gewonnen werden. Die Erucasäure (cis-13-Docosensäure) kommt als Glycerinester in Senf-, Traubenkern-, Dorschleber- und Cruciferenölen, insbesondere in Rapsöl vor. Die dazu stereoisomere Brassidinsäure (trans-13-Docosensäure) kommt in der Natur nicht vor, ist jedoch bei Erwärmen mit salpetriger Säure aus der Erucasäure erhältlich. Die Nervonsäure (Selacholeinsäure, cis-15-Tetracosensäure) kommt in Haifischleberölen, Sphingomyelinen und Cerebrosiden, insbesondere Nervon vor.

Zur Herstellung der erfindungsgemäßen Substanzen können diese Fettsäuren der allgemeinen Formel (VIII):

$$R - OOH \qquad (VIII)$$

in der R ein Erucyl-, Brassidyl- oder Nervonylrest ist, durch Reduktion nach bekannten Methoden, vorzugsweise mit $LiAlH_4$ in die entsprechenden Alkohole der allgemeinen Formel (IX):

$$R - OH \qquad (IX)$$

überführt werden. Diese Alkohole können dann nach ebenfalls bekannten Verfahren in die erfindungsgemäßen Substanzen überführt werden.

Die Herstellung von erfindungsgemäßen Verbindungen (I), bei denen X ein Sauerstoffatom und A eine Einfachbindung bedeutet, können z.B. nach den in DE 27 52 125, EP-A 0 108 565, DE 36 41 491, DE 36 41 379 , DE 36 41 377 und DE 40 13 632 offenbarten Verfahren oder gemäß der darin zitierten Literatur hergestellt werden.

Vorzugsweise wird dabei der Alkohol R-OH bei dem R einen Erucyl-, Brassidyl- oder Nervonyl-Rest darstellt, direkt durch Phosphorylierung in die entsprechenden Alkylphosphoaminderivate, insbesondere in die entsprechenden Alkylphosphocholine überführt. Hierzu kann man z.B. den Alkohol (IX) mit einer Verbindung der allgemeinen Formel (X):

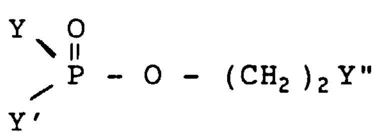

(X)

umsetzen, wobei Y, Y' und Y" Halogenreste darstellen (z.B. kann es sich bei der Verbindung (X) um 2-Bromethylphosphordichlorid handeln). Aus dieser Reaktion und anschließender Aufarbeitung durch Hydrolyse resultiert eine

Verbindung mit der allgemeinen Formel (XI):

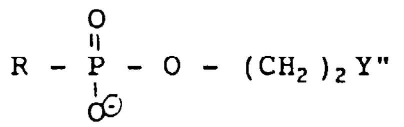

(XI)

bei der R und Y" die oben genannten Bedeutungen besitzen. Der letzte Schritt der Reaktion umfaßt die Umsetzung dieser Verbindung (XI) mit einem Amin der Formel N - $R_1$,$R_2$,$R_3$ bzw. einem quarternären Ammoniumsalz der Formel $HN^+R_1$,$R_2$,$R_3Y^-$, wobei $R_1$, $R_2$ und $R_3$ die in Anspruch 1 angegebenen Bedeutungen besitzen. Beispiele für erfindungsgemäß geeignete tertiäre Amine sind etwa Trimethylamin, Dimethylethylamin, Diethylmethylamin, Triethylamin, N.N-Dimethyl-N-propylamin, N.N-Dimethyl-N-iso-propylamin, N-Cyclopropyl-N.N-dimethylamin, N-Allyl-N.N-dimethylamin, N-Ethyl-N-methyl-N-propylamin, N-Butyl-N.N-dimethylamin, N.N-Dimethyl-N-hydroxyethylamin, N.N-Dihydroxyethyl-N-methylamin, N-Cyclobutyl-N.N-dimethylamin, N-Methylpyrrolidin, N-Ethylpyrrolidin, N-Methylmorpholin und dgl..

Andererseits kann man zur Herstellung der erfindungsgemäßen Substanzen den Alkohol (IX) mit Phosphoroxytrichlorid ($POCl_3$) umsetzen. Bei nachfolgender Hydrolyse erhält man ein Produkt der allgemeinen Formel (XII)

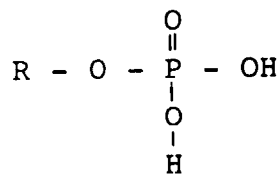

(XII)

Dieses Produkt wiederum kann mit einem tertiären Ammoniumsalz der Formel $HOCH_2$-$CH_2$-$N^+$-$R_1$,$R_2$,$R_3$ zu einer erfindungsgemäßen Substanz der allgemeinen Formel I umgesetzt werden. Die detaillierten Reaktionsbedingungen sind dabei aus den oben genannten Literaturstellen zu entnehmen.

Das bevorzugte Verfahren zur Herstellung der erfindungsgemäßen Substanzen ist die Umsetzung des Alkohols (IX) mit $POCl_3$ unter Bildung des entsprechenden Phosphorsäuredichlorids (XIII)

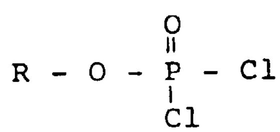

(XIII).

Diese Verbindung wird mit einem Ethanolamin oder einem entsprechend substituierten Ethanolamin zu einem heterozyklischen 5-Ring umgesetzt (XIV)

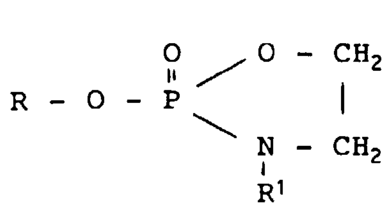

(XIV)

$R^1$ = H, $CH_3$, $CH_2CH_3$ usw.

Die Öffnung des Ringes unter sauren Bedingungen führt zu den entsprechenden Phosphoethanolaminen, die durch Alkylierung in die gewünschten peralkylierten Verbindungen der allgemeinen Formel I, u.a. in Phosphocholin umgewandelt werden können. Dieses Verfahren ist detailliert in H. Eibl (Proc.Natl.Acad. Sci. USA <u>75</u> (1978), 4074-4077) beschrieben.

Die Herstellung von erfindungsgemäßen Verbindungen der allgemeinen Formel (I), wo A eine Gruppe mit den Formeln (II), (IV), (V), (VI) und (VII)

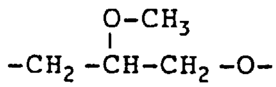

(II),

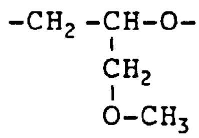

(IV),

$$-CH_2-CH_2-CH_2-O- \qquad (V),$$

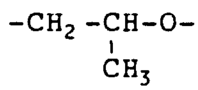

(VI)

oder

$$-CH_2-CH_2-O \qquad (VII)$$

ist, erfolgt ebenfalls nach bekannten Verfahren. Ein Beispiel hierfür ist neben den bereits oben zitierten Methoden insbesondere die DE 32 39 817. Ferner können die Alkohole der Formel (IX) auch über ihre Mesylate in die entsprechenden Alkylglycerine oder anderen Derivate umgewandelt werden, deren Phosphorylierung dann zu erfindungsgemäßen Substanzen führt.

Die Herstellung von Verbindungen, bei denen X Schwefel ist, kann gemäß Bosies et al. (Lipids 22 (1987), 947-951) durch eine mehrstufige Reaktion aus Glycerin und einem Thiol der allgemeinen Formel (XV):

$$R-SH \qquad (XV)$$

erfolgen, wobei R ein Erucyl-, Brassidyl- oder Nervonylrest ist. Andererseits kann die Phosphorylierung des Thiols auch durch die oben zitierten Phosphorylierungsmethoden erfolgen.

Die Herstellung von Verbindungen der Formel (I), bei denen A eine Gruppe mit der Formel (III)

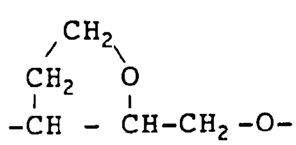

(III)

darstellt, erfolgt gemäß Houlihan et al. (Lipids 22 (1987), 884-890) ausgehend von der kommerziell erhältlichen 2-Furancarbonsäure. Andererseits kann der Alkohol als Grundkörper jedoch auch nach den oben zitierten Methoden phosphoryliert und weiter umgesetzt werden.

Die erfindungsgemäßen Substanzen haben sich sehr gut als Arzneimittel geeignet erwiesen. Ein Gegenstand der vorliegenden Erfindung ist daher auch ein Arzneimittel, das als Wirkstoff eine oder mehrere der erfindungsgemäßen

Substanzen, gegebenenfalls zusammen mit pharmazeutisch üblichen Träger-, Hilfs-, Füll- und Verdünnungsmitteln enthält. Vorzugsweise enthält das erfindungsgemäße Arzneimittel als Wirkstoff Erucyl-, Brassidyl- oder Nervonylphosphocholin, besonders bevorzugt Erucylphosphocholin.

Die erfindungsgemäßen Substanzen können auch in Kombination mit Alkylglycerinen der allgemeinen Formel (XVI)

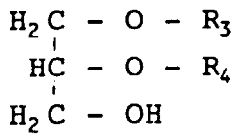

(XVI),

verwendet werden, in der einer der Reste $R^3$ und $R^4$ eine Alkylgruppe mit 2 bis 12 C-Atomen und der andere Rest ein H-Atom bedeutet. Vorzugsweise enthält ein derartiges Arzneimittel ein Alkylglycerin-Gemisch aus Nonyl- bzw. Octylglycerin, Hexyl- bzw. Pentylglycerin und Propyl- bzw. Ethylglycerin sowie Wasser. Derartige Arzneimittelkombinationen von Alkylglycerinen und Phospholipiden und die bevorzugten Gehalte der einzelnen Wirkstoffe sind in der DE-OS 36 41 379 offenbart. Die Arzneimittel, die eine erfindungsgemäße Substanz in Kombination mit mindestens einem Alkylglycerin enthalten, sind insbesondere zur topischen Applikation geeignet.

Überraschenderweise besitzen die erfindungsgemäßen Substanzen, insbesondere die Erucyl- und Nervonylderivate keine hämolytischen Eigenschaften, wie sie bei anderen Lysolecitinen beobachtet wurden. Deshalb können diese Substanzen in physiologischen Lösungen (Kochsalz-, Ringer- etc.) aufgenommen und intravenös appliziert werden. Noch überraschender ist aber die Tatsache, daß diese Substanzen gegenüber Hexadecylphosphocholin eine deutlich bessere Wirkung besitzen.

Besonders geeignet haben sich die erfindungsgemäßen Arzneimittel zur Bekämpfung von Tumoren erwiesen. So wird mit Erucylphosphocholin eine gegenüber Hexadecylphosphocholin in gleichen Mengen erheblich verbesserte Kontrolle des Tumorwachstums bei Methylnitroharnstoff induzierten Mammakarzinomen erzielt. Die erfindungsgemäßen Arzneimittel sind weiterhin zur Bekämpfung von Protozoen- und Pilzerkrankungen, insbesondere der Leishmaniasis geeignet. Ferner eignen sich die erfindungsgemäßen Arzneimittel auch zur Therapie von Autoimmunerkrankungen, insbesondere von Multipler Sklerose. Überdies ist eine Therapie von Knochenmarkschädigungen möglich, die durch Behandlung mit Zytostatika und anderen knochenmarkschädigenden Wirkstoffen aufgetreten ist.

Weiterhin ein Gegenstand der vorliegenden Erfindung ist auch ein Verfahren zur Herstellung eines erfindungsgemäßen Arzneimittels, das insbesondere als Antitumormittel, Mittel zur Bekämpfung von Protozoen- und Pilzerkrankungen, insbesondere von Leishmaniasis, als Mittel zur Therapie von Autoimmunerkrankungen, insbesondere von Multipler Sklerose sowie als Mittel zur Therapie von Knochenmarkschädigungen formuliert ist.

Durch Verabreichung des erfindungsgemäßen Arzneimittels werden Verfahren zur Bekämpfung von Tumoren, Protozoen- und Pilzerkrankungen, Autoimmunerkrankungen oder Knochenmarkschädigungen bereitgestellt. Vorzugsweise wird dabei das Arzneimittel intravenös verabreicht. Es ist jedoch ebenfalls eine subkutane oder topische Verabreichung des erfindungsgemäßen Arzneimittels möglich.

Die vorliegende Erfindung soll durch die folgenden Beispiele weiter verdeutlicht werden.

Beispiele

Gruppe I: Erucylreste und Brassidylreste

Beispiel 1

Erucyl-Phosphocholin

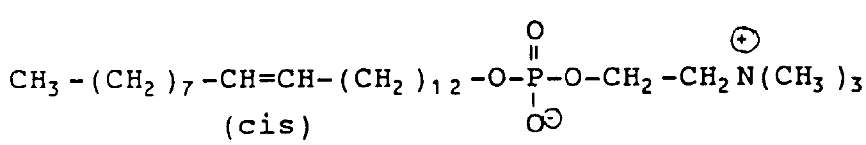

$C_{27}H_{56}NO_4P$; 489.722

| | | | | |
|---|---|---|---|---|
| ber.(%): | C, 66.22 | H, 11.53 | N, 2.86 | P, 6.33 |
| gef.(%): | 65.98 | 11.45 | 2.69 | 6.21 |

Die Herstellung der Verbindungen gemäß den Beispielen 1 bis 19 und 33 bis 35 erfolgt gemäß Methoden, wie sie z.B. in DE 27 52 125, DE 36 41 379, DE 36 41 491, DE 36 41 377 und DE 40 13 632 oder der darin zitierten Literatur beschrieben sind.

Beispiel 2

Brassidyl-Phosphocholin

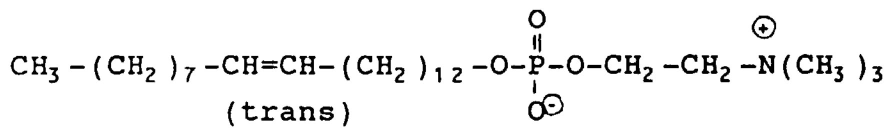

$C_{27}H_{56}NO_4P$; 489.722

| | | | | |
|---|---|---|---|---|
| gef.(%): | C, 66.04 | H, 11.50 | N, 2.54 | P, 6.17 |

Beispiel 3

Erucyl-phospho-(N.N-dimethyl-N-ethyl)-ethanolamin

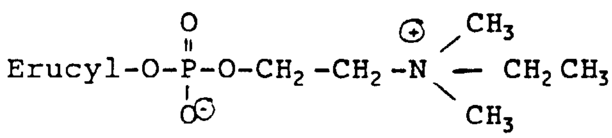

$C_{28}H_{58}NO_4P$, 503.749

| | | | | |
|---|---|---|---|---|
| ber.(%): | C, 66.76 | H, 11.61 | N, 2.78 | P, 6.15 |
| gef.(%): | 66.51 | 11.53 | 2.69 | 6.01 |

Beispiel 4

Brassidyl-phospho-(N.N-dimethyl-N-ethyl)-ethanolamin $C_{28}H_{58}NO_4P$; 503.749

Beispiel 5

Erucyl-phospho-(N.N-diethyl-N-methyl)-ethanolamin

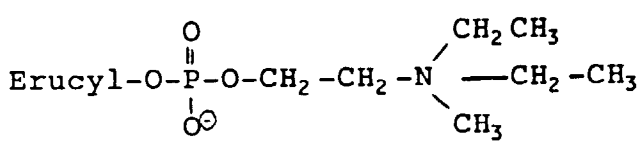

$C_{29}H_{60}NO_4P$; 517.776

Beispiel 6

Brassidyl-phospho-(N.N-diethyl-N-methyl)-ethanolamin $C_{29}H_{60}NO_4P$; 517.776

Beispiel 7

Erucyl-phospho-(N.N.N-triethyl)-ethanolamin

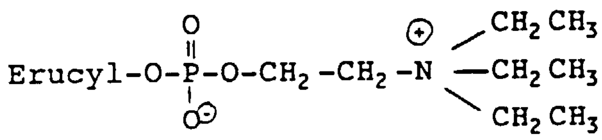

$C_{30}H_{62}NO_4P$; 531.803

Beispiel 8

Erucyl-phospho-(N.N-dimethyl-N-propyl)-ethanolamin

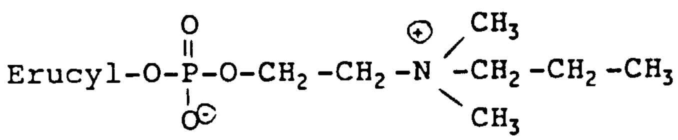

$C_{29}H_{60}NO_4P$; 517.776

Beispiel 9

Erucyl-phospho-(N.N-dimethyl-N-isopropyl)-ethanolamin

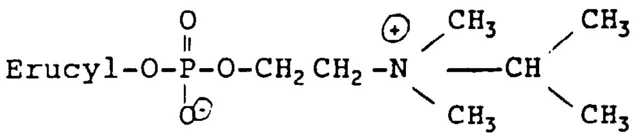

$C_{29}H_{60}NO_4P$; 517.776

Beispiel 10

Erucyl-phospho-(N-cyclopropyl-N.N-dimethyl)-ethanolamin

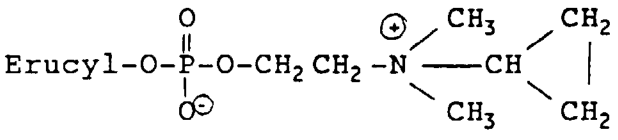

$C_{29}H_{58}NO_4P$; 515.760

Beispiel 11

Erucyl-phospho-(N-allyl-N.N-dimethyl)-ethanolamin

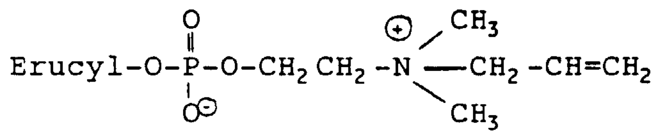

$C_{29}H_{58}NO_4P$; 515.760

Beispiel 12

Erucyl-phospho-(N-ethyl-N-methyl-N-propyl)-ethanolamin

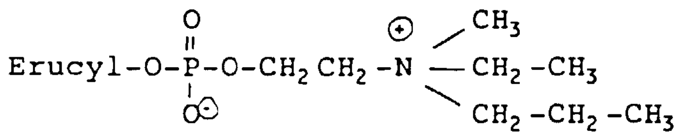

$C_{30}H_{62}NO_4P$; 531.803

Beispiel 13

Erucyl-phospho-(N-butyl-N.N-dimethyl)-ethanolamin

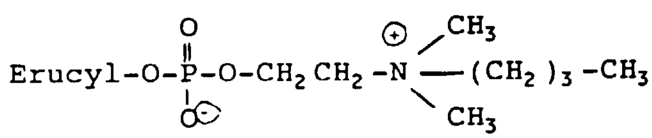

$C_{30}H_{62}NO_4P$; 530.803

Beispiel 14

Erucyl-phospho-(N.N-dimethyl-N-hydroxyethyl)-ethanolamin

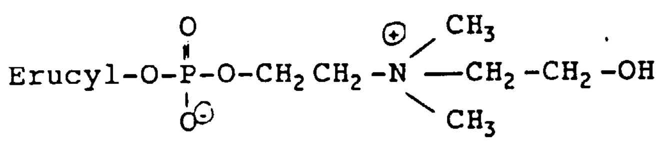

$C_{28}H_{58}NO_5P$; 519.748

Beispiel 15

Erucyl-phospho-(N.N-dihydroxyethyl-N-methyl)-ethanolamin

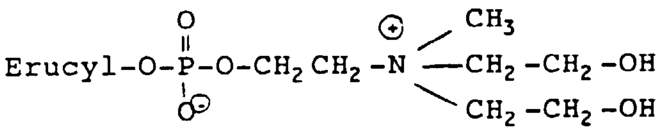

$C_{29}H_{60}NO_6P$; 549.774

Beispiel 16

Erucyl-phospho-(N-cyclobutyl-N.N-dimethyl)-ethanolamin

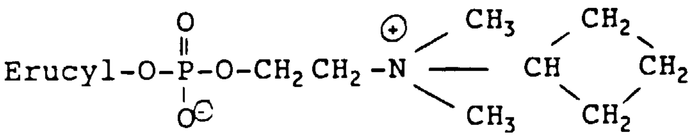

$C_{30}H_{60}NO_4P$; 529.787

Beispiel 17

Erucyl-phosphorsäure-(N-methyl)-pyrrolidinoethylester

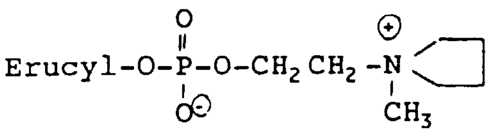

$C_{29}H_{58}NO_4P$; 515.760

Beispiel 18

Erucyl-phosphorsäure-(N-ethyl)-pyrrolidinoethylester

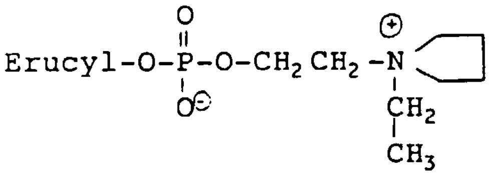

$C_{30}H_{60}NO_4P$; 529.787

Beispiel 19

Erucyl-phosphorsäure-(N-methyl)-morpholino-ethylester

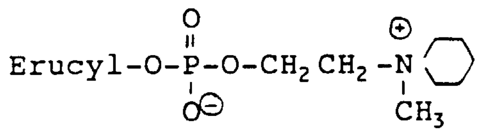

$C_{30}H_{60}NO_4P$; 529.787

Beispiel 20

1-Erucyl-2-methyl-rac-glycero-3-phosphocholin

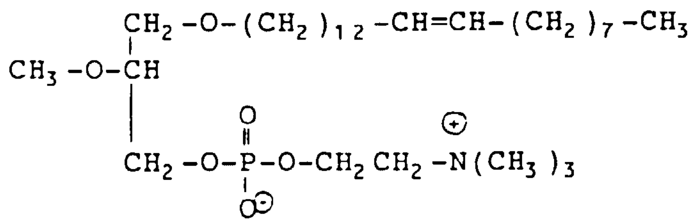

$C_{31}H_{64}NO_6P$; 577.828

Die Herstellung der Verbindungen gemäß den Beispielen 20 bis 22 und 27 bis 32 erfolgt wie z.B. in der DE 32 39 817 und der dort zitierten Literatur oder wie in Beispiel 1 beschrieben.

Beispiel 21

1-Erucyl-2-methyl-sn-glycero-3-phosphocholin

Beispiel 22

3-Erucyl-2-methyl-sn-glycero-1-phosphocholin

Alle Modifikationen bezüglich der Aminogruppe sowie der Austausch des Erucylrests gegen einen Brassidylrest, die in den Beispielen 1 bis 19 beschrieben worden sind, können auch auf das Grundgerüst 1-Erucyl-2-methyl-glycerin übertragen werden, mit der gleichen Methodologie und vergleichbaren Ausbeuten.

Beispiel 23

1-Erucylmercapto-2-methoxymethylpropyl-2'-(N.N.N-trimethyl)-ammonio-ethylphosphat

$C_{32}H_{66}NO_5PS$, 607.916

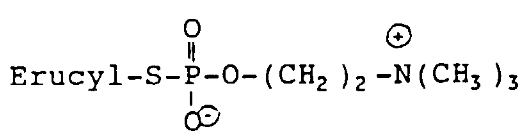

| | | | | |
|---|---|---|---|---|
| ber.(%): | C, 63.23 | H, 10.94 | N, 2.30 | P, 5.10 |
| gef.(%): | 62.95 | 10.89 | 2.26 | 4.98 |

Die Schwefel enthaltenden Verbindungen können nach Bosies et al. (Lipids, 22 (1987) 947-951) hergestellt werden. Das entsprechende Thiol wurde jedoch nach den in Beispiel 1 zitierten Phosphorylierungsmethoden phosphoryliert und weiter umgesetzt.

Auch hier können die Modifikationen 1 bis 19 eingeführt werden, ausgehend von dem entsprechenden Alkohol.

Beispiel 24

1-Erucylmercapto-2-methoxymethylpropyl-2'-(N.N-dihydroxyethyl-N-methyl)-ammonio-ethylphosphat
$C_{34}H_{70}NO_7PS$, 667.968

Beispiel 25

Weiter können nach Houlihan et al. (Lipids, 22 (1987) 884-890) folgende Wirkstoffe, die Erucyl- oder Brassidyl-Reste enthalten, hergestellt werden, die auf folgendem Grundgerüst aufgebaut sind: (±) -2-{Hydroxy[tetrahydro-2-(alkyl)-methylfuran-2-yl]methoxyphosphinyloxy}-N.N.N-trimethylethaniminiumhydroxid. Der Alkohol als Grundkörper wurde jedoch nach den in Beispiel 1 zitierten Methoden phosphoryliert und weiter umgesetzt.

Erucyl als Alkylrest $C_{32}H_{64}NO_6P$; 589.839

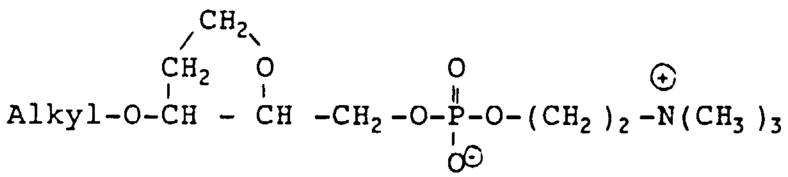

Beispiel 26

Brassidyl als Alkylrest $C_{32}H_{64}NO_6P$; 589.839

Beispiel 27

1-Erucyl-3-methyl-rac-glycero-3-phosphocholin

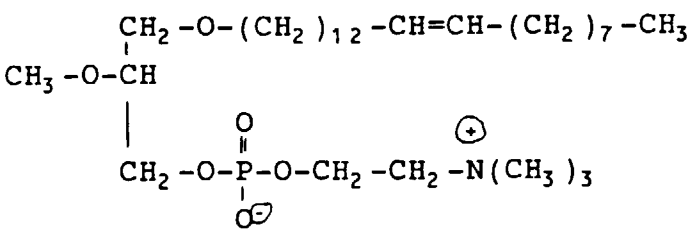

$C_{31}H_{64}NO_6P$; 577.828

| | | | | |
|---|---|---|---|---|
| ber.(%): | C, 64.44 | H, 11.17 | N, 2.42 | P, 5.36 |
| gef.(%): | 64.29 | 11.11 | 2.39 | 5.31 |

Beispiel 28

1-Erucyl-3-methyl-sn-glycero-3-phosphocholin

Beispiel 29

3-Erucyl-1-methyl-sn-glycero-2-phosphocholin

Beispiel 30

1-Erucyl-propandiol-(1,3)-phosphocholin

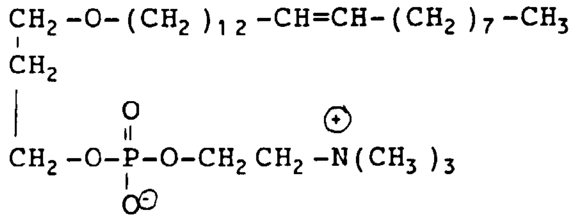

$C_{30}H_{62}NO_5P$; 547.802

| | | | | |
|---|---|---|---|---|
| ber.(%):<br>gef.(%): | C, 65.78<br>65.61 | H, 11.41<br>11.35 | N, 2.56<br>2.49 | P, 5.65<br>5.58 |

Beispiel 31

1-Erucyl-propandiol-(1,2)-phosphocholin
$C_{30}H_{62}NO_5P$; 547.802

Beispiel 32

Erucyl-glykol-phosphocholin

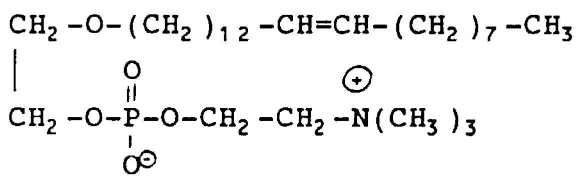

$C_{29}H_{60}NO_5P$; 533.775

| | | | | |
|---|---|---|---|---|
| ber.(%):<br>gef.(%): | C, 65.26<br>65.17 | H, 11.33<br>11.26 | N, 2.62<br>2.57 | P, 5.80<br>5.72 |

Gruppe II: Nervonylreste

Alle Verbindungen aus Gruppe I (Beispiel 1 bis 32) können durch analoge Verfahren auch mit Nervonylresten hergestellt werden.

Beispiel 33

Nervonyl-Phosphocholin (cis-15-Tetracosenyl-PC)

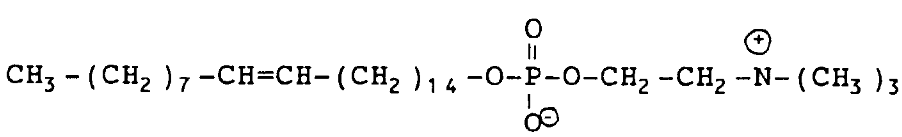

$C_{29}H_{60}NO_4P$; 517.776

| | | | | |
|---|---|---|---|---|
| ber.: | C, 67.27 | H, 11.68 | N, 2.71 | P, 5.98 |

(fortgesetzt)

| | | | | |
|---|---|---|---|---|
| | 67.13 | 11.59 | 2.64 | 5.68 |

Beispiel 34

Nervonyl-phospho-(N.N-dimethyl-N-ethyl)-ethanolamin

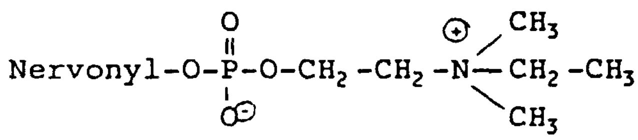

$C_{30}H_{62}NO_4P$; 531.803

Beispiel 35

Nervonyl-phospho-(N.N-diethyl-N-methyl)-ethanolamin

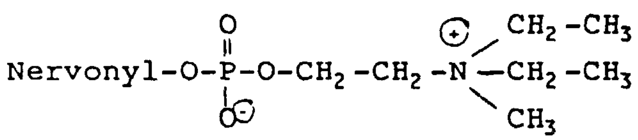

$C_{31}H_{64}NO_4P$; 545.830

Nervonylderivate, die zu den Verbindungen gemäß den Beispielen 7 bis 26 analog sind, werden nach den jeweils dort beschriebenen Verfahren hergestellt.

Anwendungsbeispiel

Die Herstellung einer Hexadecylphosphocholin-Formulierung in Liposomen erfolgt gemäß DE 40 26 136.0. Hexadecylphosphocholin (12 mmol), Cholesterin (15 mmol) und DPPG (3 mmol) werden in 200 ml 2-Propanol unter Erwärmen gelöst. Dann wird das Lösungsmittel im Vakuum abgezogen und der feinverteilte Lipidfilm mit 300 ml Phosphatpufferlösung, pH 7,0 versetzt. Anschließend wird das Gemisch unter langsamer Rotation 60 Minuten bei 40°C gehalten.

Anschließend wird die erhaltene Lipidsuspension in die Druckzelle der French Press überführt und bei 740 MPa ausgepreßt und dieser Vorgang dreimal wiederholt. Die gebildete Liposomendispersion wird dann 30 Minuten bei 27.000 g und 5°C zentrifugiert und der Überstand gewonnen.

Gleichzeitig wurde eine Erucylphosphocholin-Formulierung in physiologischer NaCl-Lösung hergestellt.

Methylnitroharnstoff-induzierte Mammakarzinome in der Ratte werden mit den wie oben hergestellten Formulierungen in einer Menge behandelt, die den angegebenen Konzentrationen von Hexadecylphosphocholin bzw. Erucylphosphocholin pro kg Ratte als Tagesdosis entspricht. Nach 4 Wochen werden die Tumorgewichte von unbehandelten Kontrolltieren auf 100 % gesetzt. Dieser Wert entspricht ungehindertem Tumorwachstum. Die Tumortiere in der behandelten Gruppe erreichen Werte zwischen 0 und 100 % im Vergleich zur Kontrollgruppe, wie in Tabelle 1 angegeben.

Tabelle 1

| Wirkstoffe | Formulierung | Menge/Tag | Wirkung* |
|---|---|---|---|
| Hexadecylphosphocholin | Liposomen | 30 µmol | < 10 % |
| | | 10 µmol | ~ 90 % |
| Erucylphosphocholin | in phys. NaCl-Lös. | 10 µmol | < 10 % |
| | | 6 µmol | < 10 % |

* Restgewicht des Tumors in % bezüglich der unbehandelten Kontrolle

Tabelle 1 (fortgesetzt)

| Wirkstoffe | Formulierung | Menge/Tag | Wirkung* |
|---|---|---|---|
| | | 3 µmol | < 10 % |

* Restgewicht des Tumors in % bezüglich der unbehandelten Kontrolle

**Patentansprüche**

**Patentansprüche für folgende Vertragsstaaten : AT, BE, CH, LI, DE, DK, FR, GB, IT, LU, MC, NL, PT, SE**

**1.** Verbindungen der allgemeinen Formel I

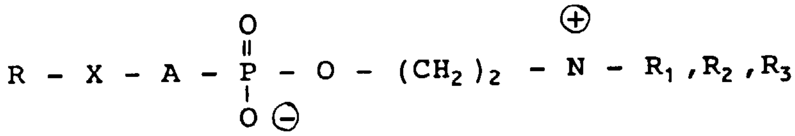

(I)

in der R einen Erucyl-, Brassidyl- oder Nervonyl-Rest,
$R_1$, $R_2$ und $R_3$ unabhängig voneinander je einen geradkettigen, verzweigten, cyclischen gesättigten oder ungesättigten Alkylrest mit 1 bis 4 C-Atomen, der auch eine OH-Gruppe enthalten kann und wobei zwei dieser Reste auch zu einem Ring verbunden sein können,
A eine Einfachbindung oder eine der Gruppen mit den Formeln

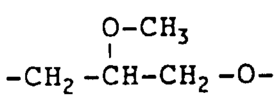

(II),

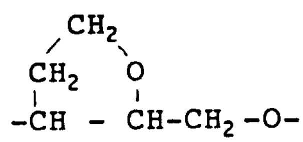

(III),

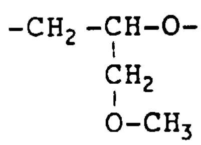

(IV),

$$-CH_2-CH_2-CH_2-O- \quad (V),$$

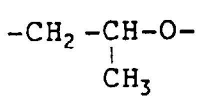

(VI)

oder

$$-CH_2-CH_2-O \quad (VII)$$

ist,

wobei die Gruppen (II) bis (VII) eine solche Orientierung besitzen, daß das Sauerstoffatom an das Phosphoratom der Verbindung (I) gebunden ist,
und X ein Sauerstoffatom ist, wenn A eine Einfachbindung ist oder ein Sauerstoff- oder Schwefelatom bedeutet, wenn A eine der Gruppen (II) bis (VII) ist.

**2.** Verbindungen nach Anspruch 1,
**dadurch gekennzeichnet**,
daß X ein Sauerstoffatom bedeutet.

**3.** Verbindungen nach Anspruch 1 oder 2,
**dadurch gekennzeichnet,**
daß A eine Einfachbindung bedeutet.

**4.** Verbindungen nach den Ansprüchen 1 bis 3,
**dadurch gekennzeichnet,**
daß $R_1$, $R_2$ und $R_3$ jeweils Methylgruppen sind.

**5.** Erucylphosphocholin.

**6.** Verfahren zur Herstellung einer Verbindung nach einem der Ansprüche 1 bis 5,
**dadurch gekennzeichnet,**
daß man eine Carbonsäure der allgemeinen Formel

$$R - OOH \qquad (VIII)$$

in der R die in Anspruch 1 angegebene Bedeutung hat, mit $LiAlH_4$ zum entsprechenden Alkohol mit der allgemeinen Formel

$$R - OH \qquad (IX)$$

reduziert und dann auf an sich bekannte Weise zu einer Verbindung mit der allgemeinen Formel (I) umsetzt.

**7.** Arzneimittel,
**dadurch gekennzeichnet,**
daß es als Wirkstoff eine oder mehrere Verbindungen nach einem der Ansprüche 1 bis 5, gegebenenfalls zusammen mit pharmazeutisch üblichen Träger-, Hilfs-, Füll- und Verdünnungsmitteln enthält.

**8.** Arzneimittel nach Anspruch 7,
**dadurch gekennzeichnet,**
daß es als Wirkstoff Erucyl-, Brassidyl- oder Nervonylphosphocholin enthält.

**9.** Arzneimittel nach Anspruch 8,
**dadurch gekennzeichnet**,
daß es als Wirkstoff Erucylphosphocholin enthält.

**10.** Arzneimittel nach einem der Ansprüche 7 bis 9,
**dadurch gekennzeichnet**,
daß es den Wirkstoff in einer physiologischen Lösung enthält.

**11.** Arzneimittel nach einem der Ansprüche 7 bis 10,
**dadurch gekennzeichnet**,
daß es zusätzlich mindestens ein Alkylglycerin der allgemeinen Formel (XVI)

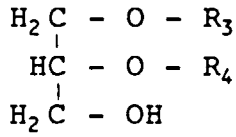

(XVI),

in der einer der Reste $R_3$ und $R_4$ eine Alkylgruppe mit 2 bis 12 C-Atomen und der andere Rest ein H-Atom bedeutet, enthält.

**12.** Arzneimittel nach Anspruch 11,
**dadurch gekennzeichnet**,
daß es ein Alkylglycerin-Gemisch enthält aus Nonyl- bzw. Octylglycerin, Hexyl- bzw. Pentylglycerin und Propyl- bzw. Ethylglycerin und Wasser.

**13.** Arzneimittel nach Anspruch 7 zur intravenösen Verabreichung.

**14.** Arzneimittel nach Anspruch 7 zur subkutanen Verabreichung.

**15.** Arzneimittel nach Anspruch 7 zur topischen Verabreichung.

**16.** Verfahren zur Herstellung eines Antitumormittels,
**dadurch gekennzeichnet,**
daß man eine Formulierung herstellt, die als Wirkstoff eine oder mehrere Verbindungen nach einem der Ansprüche 1 bis 5 enthält, gegebenenfalls zusammen mit pharmazeutisch üblichen Träger-, Hilfs-, Füll- und Verdünnungsmitteln.

**17.** Verfahren zur Herstellung eines Mittels zur Bekämpfung von Protozoen- und Pilzerkrankungen, insbesondere von Leishmaniasis,
**dadurch gekennzeichnet,**
daß man eine Formulierung herstellt, die als Wirkstoff eine oder mehrere Verbindungen nach einem der Ansprüche 1 bis 5 enthält, gegebenenfalls zusammen mit pharmazeutisch üblichen Träger-, Hilfs-, Füll- und Verdünnungsmitteln.

**18.** Verfahren zur Herstellung eines Mittels zur Therapie von Autoimmunerkrankungen, insbesondere von Multipler Sklerose,
**dadurch gekennzeichnet,**
daß man eine Formulierung herstellt, die als Wirkstoff eine oder mehrere Verbindungen nach einem der Ansprüche 1 bis 5 enthält, gegebenenfalls zusammen mit pharmazeutisch üblichen Träger-, Hilfs-, Füll- und Verdünnungsmitteln.

**19.** Verfahren zur Herstellung eines Mittels zur Therapie von Knochenmarksschädigungen,
**dadurch gekennzeichnet,**
daß man eine Formulierung herstellt, die als Wirkstoff eine oder mehrere Verbindungen nach einem der Ansprüche 1 bis 5 enthält, gegebenenfalls zusammen mit pharmazeutisch üblichen Träger-, Hilfs-, Füll- und Verdünnungsmitteln.

**Patentansprüche für folgende Vertragsstaat : ES, GR**

**1.** Verfahren zur Herstellung von Verbindungen der allgemeinen Formel I

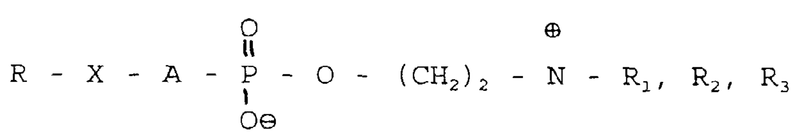

(I)

in der R einen Erucyl-, Brassidyl- oder Nervonyl-Rest,
$R_1$, $R_2$ und $R_3$ unabhängig voneinander je einen geradkettigen, verzweigten, cyclischen gesättigten oder ungesättigten Alkylrest mit 1 bis 4 C-Atomen, der auch eine OH-Gruppe enthalten kann und wobei zwei dieser Reste auch zu einem Ring verbunden sein können,
A eine Einfachbindung oder eine der Gruppen mit den Formeln

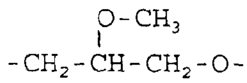 (II),

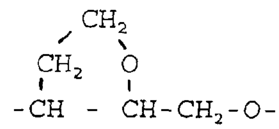 (III),

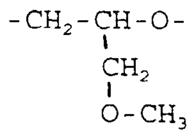 (IV),

$$-CH_2-CH_2-CH_2-O- \qquad (V),$$

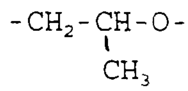 (VI)

oder

$$-CH_2-CH_2-O \qquad (VII)$$

ist,
wobei die Gruppen (II) bis (VII) eine solche Orientierung besitzen, daß das Sauerstoffatom an das Phosphoratom der Verbindung (I) gebunden ist,
und X ein Sauerstoffatom ist, wenn A eine Einfachbindung ist oder ein Sauerstoff- oder Schwefelatom bedeutet, wenn A eine der Gruppen (II) bis (VII) ist,
**dadurch gekennzeichnet,**
daß man eine Verbindung der allgemeinen Formel

RXAH

1) mit einem Halogenethylphosphordichlorid phosphoryliert und das Produkt unter Bildung eines entsprechenden Alkylphosphoamins hydrolysiert und dann in das quaternäre Ammoniumsalz überführt oder
2) mit $POCl_3$ umsetzt und

a) das erhaltene Produkt hydrolysiert und danach mit einem tertiären Ammoniumsalz der Formel $HOCH_2-CH_2N^{\oplus}-R_1,R_2,R_3$ umsetzt oder
b) das erhaltene Phosphorsäuredichlorid $R-O-POCl_2$ mit einem Ethanolamin zu einer Verbindung der allgemeinen Formel

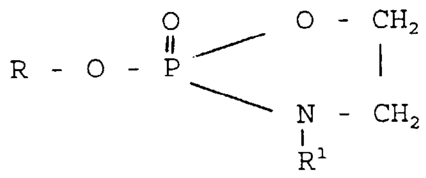

(XIV)

umsetzt, den Ring unter sauren Bedingungen öffnet und das erhaltene Phosphorethanolamin alkyliert.

2. Verfahren nach Anspruch 1,
**dadurch gekennzeichnet,**
daß X ein Sauerstoffatom bedeutet.

3. Verfahren nach Anspruch 1 oder 2,
**dadurch gekennzeichnet,**
daß A eine Einfachbindung bedeutet.

4. Verfahren nach den Ansprüchen 1 bis 3,
**dadurch gekennzeichnet,**
daß $R_1$, $R_2$ und $R_3$ jeweils Methylgruppen sind

5. Verfahren nach einem der Ansprüche 1 bis 4,
**dadurch gekennzeichnet,** daß man eine Carbonsäure der allgemeinen Formel

$$R - OOH \qquad (VIII)$$

in der R die in Anspruch 1 angegebene Bedeutung hat, mit $LiAlH_4$ zum entsprechenden Alkohol mit der allgemeinen Formel

$$R- OH \qquad (IX)$$

reduziert und dann gemäß Anspruch 1 weiter umsetzt.

6. Verfahren zur Herstellung eines Arzneimittels,
**dadurch gekennzeichnet,**
daß man als Wirkstoff eine oder mehrere nach einem der Ansprüche 1 bis 5 erhaltene Verbindungen zusammen mit pharmazeutisch üblichen Träger, Hilfs-, Füll und Verdünnungsmitteln zubereitet.

7. Verfahren nach Anspruch 6,
**dadurch gekennzeichnet,**
daß man als Wirkstoff Erucyl-, Brassidyl- oder Nervonyl-phosphocholin verwendet.

8. Verfahren nach Anspruch 6 oder 7,
**dadurch gekennzeichnet**,
daß man den Wirkstoff in einer physiologischen Lösung auflöst.

9. Verfahren nach einem der Ansprüche 6 bis 8,
**dadurch gekennzeichnet**,
daß man zusätzlich mindestens ein Alkylglycerin der allgemeinen Formel (XVI)

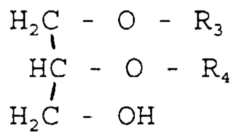

(XVI)

in der einer der Reste $R_3$ und $R_4$ eine Alkylgruppe mit 2 bis 12 C-Atomen und der andere Rest ein H-Atom bedeutet, verwendet.

**10.** Verfahren nach Anspruch 9,
**dadurch gekennzeichnet,**
daß man ein Alkylglycerin-Gemisch aus Nonyl- bzw. Octylglycerin, Hexyl- bzw. Pentylglycerin und Propyl- bzw. Ethylglycerin und Wasser verwendet.

**11.** Verfahren zur Herstellung eines Antitumormittels,
**dadurch gekennzeichnet,**
daß man eine Formulierung herstellt, die als Wirkstoff eine oder mehrere nach einem der Ansprüche 1 bis 5 hergestellte Verbindungen enthält, gegebenenfalls zusammen mit pharmazeutisch üblichen Träger-, Hilfs-, Füll- und Verdünnungsmitteln.

**12.** Verfahren zur Herstellung eines Mittels zur Bekämpfung von Protozoen- und Pilzerkrankungen, insbesondere von Leishmaniasis,
**dadurch gekennzeichnet,**
daß man eine Formulierung herstellt, die als Wirkstoff eine oder mehrere nach einem der Ansprüche 1 bis 5 hergestellte Verbindungen enthält, gegebenenfalls zusammen mit pharmazeutisch üblichen Träger-, Hilfs-, Füll- und Verdünnungsmitteln.

**13.** Verfahren zur Herstellung eines Mittels zur Therapie von Autoimmunerkrankungen, insbesondere von Multipler Sklerose,
**dadurch gekennzeichnet,**
daß man eine Formulierung herstellt, die als Wirkstoff eine oder mehrere nach einem der Ansprüche 1 bis 5 hergestellte Verbindungen enthält, gegebenenfalls zusammen mit pharmazeutisch üblichen Träger-, Hilfs-, Füll- und Verdünnungsmitteln.

**14.** Verfahren zur Herstellung eines Mittels zur Therapie von Knochenmarksschädigungen,
**dadurch gekennzeichnet,**
daß man eine Formulierung herstellt, die als Wirkstoff eine oder mehrere nach einem der Ansprüche 1 bis 5 hergestellte Verbindungen enthält, gegebenenfalls zusammen mit pharmazeutisch üblichen Träger-, Hilfs-, Füll- und Verdünnungsmitteln.

## Revendications

### Revendications pour les Etats contractants suivants : AT, BE, CH, LI, DE, DK, FR, GB, IT, LU, MC, NL, PT, SE

**1.** Composés de formule générale I

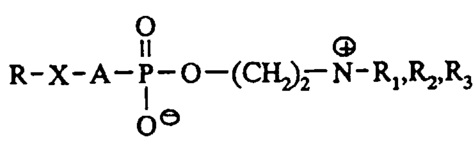

(I)

dans laquelle
R représente un reste érucyle, brassidyle ou nervonyle,

$R_1$, $R_2$ et $R_3$ représentent chacun, indépendamment les uns des autres, un reste alkyle de 1 à 4 atomes de carbone linéaire, ramifié ou cyclique, saturé ou insaturé, qui peut aussi contenir un groupe OH, deux de ces restes pouvant aussi être reliés pour former un cycle,
A représente une liaison simple ou l'un des groupes ayant les formules

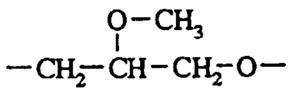 (II), 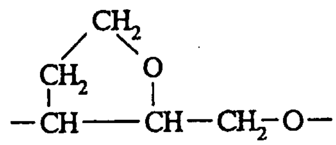 (III)

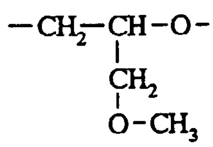 (IV),

$$-CH_2-CH_2-CH_2-O- \qquad (V),$$

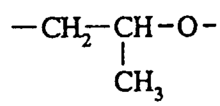 (VI)

ou

$$-CH_2-CH_2-O- \qquad (VII),$$

les groupes (II) à (VII) ayant une orientation telle que l'atome d'oxygène est lié à l'atome de phosphore du composé (I),
et X est un atome d'oxygène lorsque A est une liaison simple, ou un atome d'oxygène ou de soufre lorsque A est l'un des groupes (II) à (VII).

2. Composés selon la revendication 1, caractérisés en ce que X représente un atome d'oxygène.

3. Composés selon la revendication 1 ou 2, caractérisés en ce que A représente une liaison simple.

4. Composés selon les revendications 1 à 3, caractérisés en ce que $R_1$, $R_2$ et $R_3$ sont tous des groupes méthyle.

5. Erucylphosphocholine.

6. Procédé de préparation d'un composé selon l'une des revendications 1 à 5, caractérisé en ce que l'on réduit un acide carboxylique de formule générale

$$R-OOH \qquad (VIII)$$

dans laquelle R a la signification indiquée dans la revendication 1, avec $LiAlH_4$ pour former l'alcool correspondant de formule générale

R-OH (IX)

que l'on fait ensuite réagir de façon connue en soi pour obtenir un composé de formule générale (I).

**7.** Médicament caractérisé en ce qu'il contient comme substance active un ou plusieurs composés selon l'une des revendications 1 à 5, éventuellement avec des supports, produits auxiliaires, charges et diluants courants dans la technique pharmaceutique.

**8.** Médicament selon la revendication 7, caractérisé en ce qu'il contient comme substance active de l'érucylphosphocholine, de la brassidylphosphocholine ou de la nervonylphosphocholine.

**9.** Médicament selon la revendication 8, caractérisé en ce qu'il contient comme substance active de l'érucylphosphocholine.

**10.** Médicament selon l'une des revendications 7 à 9, caractérisé en ce qu'il contient la substance active dans une solution physiologique.

**11.** Médicament selon l'une des revendications 7 à 10, caractérisé en ce que en ce qu'il contient en outre au moins un alkylglycérol de formule générale (XVI)

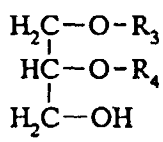

(XVI)

dans laquelle l'un des restes $R_3$ et $R_4$ représente un groupe alkyle de 2 à 12 atomes de carbone et l'autre représente un atome d'hydrogène.

**12.** Médicament selon la revendication 11, caractérisé en ce qu'il contient un mélange d'alkylglycérols constitué de nonyl- ou octyl-glycérol, d'hexyl- ou pentylglycérol et de propyl- ou éthylglycérol et d'eau.

**13.** Médicament selon la revendication 7 pour l'administration intraveineuse.

**14.** Médicament selon la revendication 7 pour l'administration sous-cutanée.

**15.** Médicament selon la revendication 7 pour l'administration topique.

**16.** Procédé de préparation d'un agent antitumoral, caractérisé en ce que l'on prépare une formulation contenant comme substance active un ou plusieurs composés selon l'une des revendications 1 à 5, éventuellement avec des supports, produits auxiliaires, charges et diluants courants dans la technique pharmaceutique.

**17.** Procédé de préparation d'un agent destiné à combattre les maladies à protozoaires et à champignons, en particulier la leishmaniose, caractérisé en ce que l'on prépare une formulation contenant comme substance active un ou plusieurs composés selon l'une des revendications 1 à 5, éventuellement avec des supports, produits auxiliaires, charges et diluants courants dans la technique pharmaceutique.

**18.** Procédé de préparation d'un agent destiné à la thérapie de maladies auto-immunes, en particulier de la sclérose en plaques, caractérisé en ce que l'on prépare une formulation contenant comme substance active un ou plusieurs composés selon l'une des revendications 1 à 5, éventuellement avec des supports, produits auxiliaires, charges et diluants courants dans la technique pharmaceutique.

**19.** Procédé de préparation d'un agent destiné à la thérapie des lésions de la moelle osseuse, caractérisé en ce que l'on prépare une formulation contenant comme substance active un ou plusieurs composés selon l'une des revendications 1 à 5, éventuellement avec des supports, produits auxiliaires, charges et diluants courants dans la technique pharmaceutique.

**Revendications pour les Etats contractants suivants : ES, GR**

**1.** Procédé de préparation de composés de formule générale I

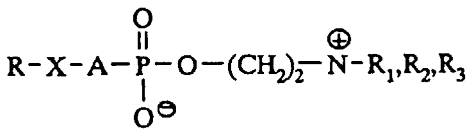

(I)

dans laquelle
R représente un reste érucyle, brassidyle ou nervonyle,
$R_1$, $R_2$ et $R_3$ représentent chacun, indépendamment les uns des autres, un reste alkyle de 1 à 4 atomes de carbone linéaire, ramifié ou cyclique, saturé ou insaturé, qui peut aussi contenir un groupe OH, deux de ces restes pouvant aussi être reliés pour former un cycle,
A représente une liaison simple ou l'un des groupes ayant les formules

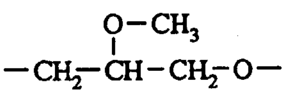

(II),

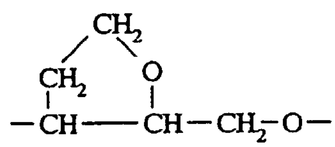

(III)

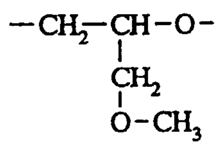

(IV),

$$-CH_2-CH_2-CH_2-O- \quad (V),$$

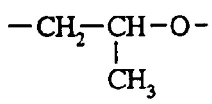

(VI)

ou

$$-CH_2-CH_2-O- \quad (VII),$$

les groupes (II) à (VII) ayant une orientation telle que l'atome d'oxygène est lié à l'atome de phosphore du composé (I),
et X est un atome d'oxygène lorsque A est une liaison simple, ou un atome d'oxygène ou de soufre lorsque A est l'un des groupes (II) à (VII),
caractérisé en ce que

1) on phosphoryle un composé de formule générale

RXAH

avec un dichlorure d'acide halogénoéthylphosphorique et on hydrolyse le produit en formant une alkylphosphoamine correspondante que l'on transforme ensuite en le sel d'ammonium quaternaire ou
2) on fait réagir le composé de formule générale

RXAH

avec $POCl_3$ et

a) on hydrolyse le produit obtenu que l'on fait ensuite réagir avec un sel d'ammonium tertiaire de formule $HOCH_2\text{-}CH_2N^{\oplus}\text{-}R_1,R_2R_3$, ou
b) on fait réagir le dichlorure d'acide phosphorique $R\text{-}O\text{-}POCl_2$ obtenu avec une éthanolamine pour former un composé de formule générale

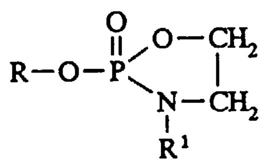

(XIV),

on ouvre le cycle dans des conditions acides et on alkyle la phosphoréthanolamine obtenue.

**2.** Procédé selon la revendication 1, caractérisé en ce que X représente un atome d'oxygène.

**3.** Procédé selon la revendication 1 ou 2, caractérisé en ce que A représente une liaison simple.

**4.** Procédé selon les revendications 1 à 3, caractérisé en ce que $R_1$, $R_2$ et $R_3$ sont tous des groupes méthyle.

**5.** Procédé selon l'une des revendications 1 à 4, caractérisé en ce que l'on réduit un acide carboxylique de formule générale

R-OOH (VIII)

dans laquelle R a la signification indiquée dans la revendication 1, avec $LiAlH_4$ pour former l'alcool correspondant de formule générale

R-OH (IX)

que l'on fait ensuite réagir selon la revendication 1.

**6.** Procédé de préparation d'un médicament, caractérisé en ce que l'on formule comme substance active un ou plusieurs composés obtenus selon l'une des revendications 1 à 5 avec des supports, produits auxiliaires, charges et diluants courants dans la technique pharmaceutique.

**7.** Procédé selon la revendication 6, caractérisé en ce que l'on utilise comme substance active de l'érucylphosphocholine, de la brassidylphosphocholine ou de la nervonylphosphocholine.

**8.** Procédé selon la revendication 7 ou 8, caractérisé en ce que l'on dissout la substance active dans une solution physiologique.

**9.** Procédé selon l'une des revendications 6 à 8, caractérisé en ce que l'on utilise en outre au moins un alkylglycérol

de formule générale (XVI)

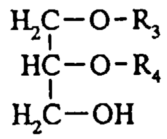

(XVI)

dans laquelle l'un des restes $R_3$ et $R_4$ représente un groupe alkyle de 2 à 12 atomes de carbone et l'autre représente un atome d'hydrogène.

**10.** Procédé selon la revendication 9, caractérisé en ce que l'on utilise un mélange d'alkylglycérols constitué de nonyl- ou octyl-glycérol, d'hexyl- ou pentylglycérol et de propyl- ou éthylglycérol et d'eau.

**11.** Procédé de préparation d'un agent antitumoral, caractérisé en ce que l'on prépare une formulation contenant comme substance active un ou plusieurs composés préparés selon l'une des revendications 1 à 5, éventuellement avec des supports, produits auxiliaires, charges et diluants courants dans la technique pharmaceutique.

**12.** Procédé de préparation d'un agent destiné à combattre les maladies à protozoaires et à champignons, en particulier la leishmaniose, caractérisé en ce que l'on prépare une formulation contenant comme substance active un ou plusieurs composés préparés selon l'une des revendications 1 à 5, éventuellement avec des supports, produits auxiliaires, charges et diluants courants dans la technique pharmaceutique.

**13.** Procédé de préparation d'un agent destiné à la thérapie de maladies auto-immunes, en particulier de la sclérose en plaques, caractérisé en ce que l'on prépare une formulation contenant comme substance active un ou plusieurs composés préparés selon l'une des revendications 1 à 5, éventuellement avec des supports, produits auxiliaires, charges et diluants courants dans la technique pharmaceutique.

**14.** Procédé de préparation d'un agent destiné à la thérapie des lésions de la moelle osseuse, caractérisé en ce que l'on prépare une formulation contenant comme substance active un ou plusieurs composés préparés selon l'une des revendications 1 à 5, éventuellement avec des supports, produits auxiliaires, charges et diluants courants dans la technique pharmaceutique.

## Claims

**Claims for the following Contracting States : AT, BE, CH, LI, DE, DK, FR, GB, IT, LU, MC, NL, PT, SE**

**1.** Compounds of the general formula I

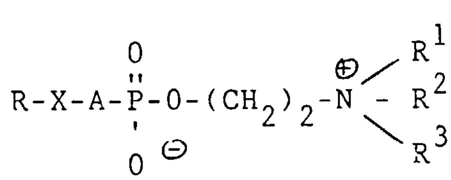

(I)

in which R is an erucyl, brassidyl or nervonyl radical, $R^1$, $R^2$ and $R^3$, independently of one another, are each straight-chained, branched, cyclic saturated or unsaturated alkyl radicals with 1 to 4 C-atoms, which can also contain an OH group, and whereby two of these radicals can also be connected to form a ring, A is a single bond or one of the groups with the formulae:

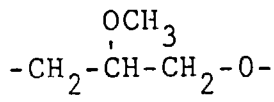

(II)

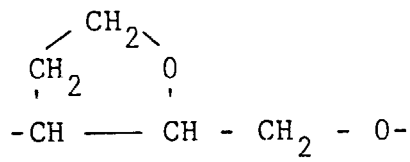

(III)

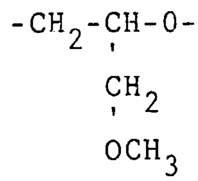

(IV)

$$-CH_2-CH_2-CH_2-O- \quad (V)$$

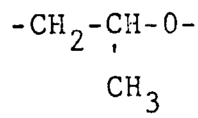

(VI)

or

$$-CH_2-CH_2-O- \quad (VII)$$

whereby the groups (II) to (VII) possess such an orientation that the oxygen atom is attached to the phosphorus atom of the compound (I), and X is an oxygen atom when A is a single bond or signifies an oxygen or sulphur atom when A is one of the groups (II) to (VII).

2. Compounds according to claim 1, characterised in that X is an oxygen atom.

3. Compounds according to claim 1 or 2,
characterised in that A signifies a single bond.

4. Compounds according to claims 1 to 3,
characterised in that $R^1$, $R^2$ and $R^3$ are each methyl groups.

5. Erucylphosphocholine.

6. Process for the preparation of a compound according to one of claims 1 to 5, characterised in that one reduces a carboxylic acid of the general formula

$$R - OOH \quad (VIII)$$

in which R has the meaning given in claim 1, with $LiAlH_4$ to the corresponding alcohol with the general formula

R - OH

which then reacts in per se known manner to a compound of the general formula (I).

**7.** Medicament, characterised in that, as active material, it contains one or more compounds according to one of claims 1 to 5, possibly together with pharmaceutically usual carrier, adjuvant, filling and dilution agents.

**8.** Medicament according to claim 7, characterised in that it contains erucyl-, brassidyl- or nervonylphosphocholine as active material.

**9.** Medicament according to claim 8, characterised in that it contains erucylphosphocholine as active material.

**10.** Medicament according to one of claims 7 to 9, characterised in that it contains the active material in a physiological solution.

**11.** Medicament according to one of claims 7 to 10, characterised in that it additionally contains at least one alkylglycerol of the general formula (XVI)

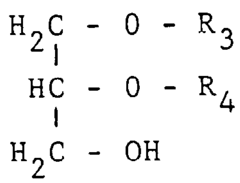

(XVI)

in which one of the radicals $R_3$ and $R_4$ signifies an alkyl group with 2 to 12 C-atoms and the other radical an H-atom.

**12.** Medicament according to claim 11, characterised in that it contains an alkylglycerol mixture of nonyl- or octylglycerol, hexyl- or pentylglycerol and propyl- or ethylglycerol and water.

**13.** Medicament according to claim 7 for intravenous administration.

**14.** Medicament according to claim 7 for subcutaneous administration.

**15.** Medicament according to claim 7 for topical administration.

**16.** Process for the preparation of an anti-tumour agent, characterised in that one prepares a formulation which, as active material, contains one or more compounds according to one of claims 1 to 5, possibly together with pharmaceutically usual carrier, adjuvant, filling and dilution agents.

**17.** Process for the preparation of an agent for the combatting of protozoal and fungal diseases, especially leishmaniasis, characterised in that one prepares a formulation which, as active material, contains one or more compounds according to one of claims 1 to 5, possibly together with pharmaceutically usual carrier, adjuvant, filling and dilution agents.

**18.** Process for the preparation of an agent for the therapy of auto-immune diseases, especially of multiple sclerosis, characterised in that one prepares a formulation which, as active material, contains one or more compounds according to one of claims 1 to 5, possibly together with pharmaceutically usual carrier, adjuvant, filling and dilution agents.

**19.** Process for the preparation of an agent for the therapy of bone marrow damage, characterised in that one prepares a formulation which, as active material, contains one or more compounds according to one of claims 1 to 5, possibly together with pharmaceutically usual carrier, adjuvant, filling and dilution agents.

**Claims for the following Contracting States : ES, GR**

1. Process for the preparation of compounds of the general formula I

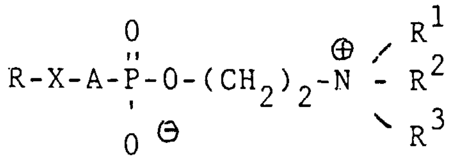

(I)

in which R is a erucyl, brassidyl or nervonyl radical, $R^1$, $R^2$ and $R^3$, independently of one another, are each straight-chained, branched, cyclic saturated or unsaturated alkyl radicals with 1 to 4 C-atoms, which can also contain an OH group, and whereby two of these radicals can also be connected to form a ring, A is a single bond or one of the groups with the formulae:

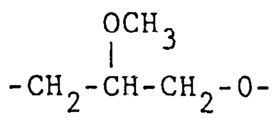

(II)

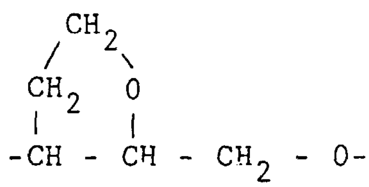

(III)

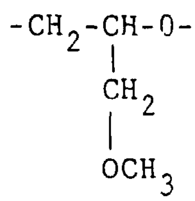

(IV)

$$-CH_2-CH_2-CH_2-O- \quad (V)$$

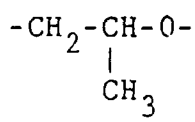

(VI)

or

$$-CH_2-CH_2-O- \quad (VII)$$

whereby the groups (II) to (VII) possess such an orientation that the oxygen atom is attached to the phosphorus atom of the compound (I), and X is an oxygen atom when A is a single bond or signifies an oxygen or sulphur atom

when A is one of the groups (II) to (VII), characterised in that a compound of the general formula

RXAH

1) is phosphorylated with a haloethyl phosphorus dichloride and the product hydrolysed with formation of a corresponding alkyl phosphoamine and then converted into the quaternary ammonium salt or
2) reacted with $POCl_3$ and

a) the product obtained is hydrolysed and thereafter reacted with a tertiary ammonium salt of the formula $HOCH_2$-$CH_2N^+$-$R_1$,$R_2$,$R_3$ or
b) the phosphoric acid dichloride R-O-$POCl_2$ obtained is reacted with an ethanolamine to give a compound of the general formula

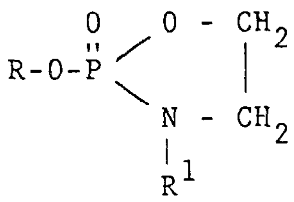

(XIV)

the ring opened under acidic conditions and the phosphorus ethanolamine obtained alkylated.

**2.** Process according to claim 1, characterised in that X signifies an oxygen atom.

**3.** Process according to claim 1 or 2, characterised in that A is a single bond.

**4.** Process according to claims 1 to 3, characterised in that $R^1$, $R^2$ and $R^3$ are each methyl groups.

**5.** Process according to one of claims 1 to 4, characterised in that one reduces a carboxylic acid of the general formula

R - OOH (VIII)

in which R has the meaning given in claim 1, with $LiAlH_4$ to give the corresponding alcohol with the general formula

R - OH (IX)

and then reacts further according to claim 1.

**6.** Process for the preparation of a medicament, characterised in that as active material, one prepares one or more of the compounds obtained according to one of claims 1 to 5, together with pharmaceutically usual carrier, adjuvant, filling and dilution agents.

**7.** Process according to claim 6, characterised in that one uses erucyl-, brassidyl- or nervonylphosphocholine as active material.

**8.** Process according to claim 6 or 7, characterised in that one dissolves the active material in a physiological solution.

**9.** Process according to one of claims 6 to 8,
characterised in that one additionally uses at least one alkylglycerol of the general formula (XVI)

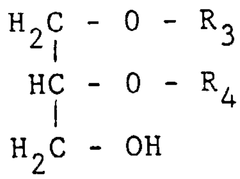

(XVI)

in which one of the radicals $R_3$ and $R_4$ signifies an alkyl group with 2 to 12 C-atoms and the other radical signifies an H-atom.

**10.** Process according to claim 9, characterised in that one uses an alkylglycerol mixture of nonyl- or octylglycerol, hexyl- or pentylglycerol and propyl- or ethylglycerol and water.

**11.** Process for the preparation of an anti-tumour agent, characterised in that one prepares a formulation which, as active material, contains one or more of the compounds prepared according to one of claims 1 to 5, possibly together with pharmaceutically usual carrier, adjuvant, filling and dilution agents.

**12.** Process for the preparation of an agent for the combatting of protozoal and fungal diseases, especially of leishmaniasis, characterised in that one prepares a formulation which, as active material, contains one or more of the compounds prepared according to one of claims 1 to 5, possibly together with pharmaceutically usual carrier, adjuvant, filling and dilution agents.

**13.** Process for the preparation of an agent for the therapy of auto-immune diseases, especially of multiple sclerosis, characterised in that one prepares a formulation which, as active material, contains one or more of the compounds prepared according to one of claims 1 to 5 and 9, possibly together with pharmaceutically usual carrier, adjuvant, filling and dilution agents.

**14.** Process for the preparation of an agent for the therapy of bone marrow damage, characterised in that one prepares a formulation which, as active material, contains one or more of the compounds prepared according to one of claims 1 to 5, possibly together with pharmaceutically usual carrier, adjuvant, filling and dilution agents.